# EUROPÄISCHE PATENTANMELDUNG

(11) **EP 4 838 127 A1**
(43) Veröffentlichungstag der Anmeldung: **11.11.2026**
(21) Anmeldenummer: 25174896.8
(22) Anmeldetag: 07.05.2025
(51) Int. Cl.: F16M 11/10, F16M 11/20, A61B 90/50

(54) **FEDERARMVERRIEGELUNG**

(71) Anmelder: Ondal Medical Systems GmbH, 36088 Hünfeld (DE)
(72) Erfinder: Oginski, Stefan, 36043 Fulda (DE)
(74) Vertreter: Maiwald GmbH

(57) **Zusammenfassung**

Vorrichtung zum Verriegeln eines Federarms (10), umfassend einen ersten Körper (16) zur Befestigung am Federarm (10), umfassend ein erstes Verriegelungselement (50), eine Aufnahme (18) zur Befestigung an einer relativ zum Federarm (10) drehbaren Komponente (12) und zum zumindest teilweise Aufnehmen des ersten Körpers (16), einen zweiten Körper (20) mit einem zweiten Verriegelungselement (24), welcher über eine Drehachse des zweiten Körpers (20) relativ zum ersten Körper (16) und zur Aufnahme (18) drehbar angeordnet ist, und ein Betätigungselement (30), welches mit einem ersten Endbereich (34) des zweiten Körpers (20), welcher sich von der Drehachse und dem zweiten Verriegelungselement (24) wegerstreckt, derart gekoppelt ist, dass das zweite Verriegelungselement (24) im aufgenommenen Zustand des ersten Körpers (16) in der Aufnahme (18) und im nicht betätigten Zustand des Betätigungselements (30) das erste Verriegelungselement (50) zumindest axial begrenzt und das zweite Verriegelungselement (24) im betätigten Zustand des Betätigungselements (30) durch eine Drehung des zweiten Körpers (20) vom ersten Verriegelungselement (50) entkoppelt ist.

## Beschreibung

### Technisches Gebiet

Die vorliegende Erfindung betrifft Vorrichtungen zum Verriegeln von Federarmen, insbesondere zum Verriegeln von Federarmen an einem Ausleger in zumindest einer Richtung senkrecht zu einer Rotationsrichtung, um einen sicheren Transport eines Endgeräts zu ermöglichen.

### Stand der Technik

In Krankenhäusern und ärztlichen Behandlungsräumen werden typischerweise Untersuchungen am Patienten mit für die Untersuchung spezifischen Geräten durchgeführt. So kann beispielsweise für Ultraschalluntersuchungen ein Ultraschallgerät auf einem fahrbaren Wagen montiert sein, um die entsprechende Untersuchung flexibel an unterschiedlichen Orten durchführen zu können. Eine Darstellung des Ultraschallbilds kann dann auf einem oder mehreren Monitoren oder Bildschirmen erfolgen. Die Bildschirme sind dabei bevorzugt flexibel aufgehängt, sodass sie über ihre Gelenke sowohl in der Höhe verstellt als auch seitlich geneigt und gedreht werden können. Typischerweise sind für die Positionierung oder Umpositionierung mehrere drehbare Komponenten vorgesehen, beispielsweise ein horizontal drehbarer Ausleger, ein horizontal drehbarer und vertikal schwenkbarer Federarm und ein drehbares und neigbares Schwenkgelenk. An einem Schwenkgelenk kann das Endgerät befestigt sein.

Entsprechend können verschiedene Geräte wie Diagnose- und/oder Überwachungsmonitore oder auch beispielsweise OP-Leuchten höhenverstellbar aufgehängt werden, um diese an die Bedürfnisse des Anwenders anzupassen und entsprechend positionieren zu können.

Beim Bewegen des Wagens kann sich das Endgerät jedoch unkontrolliert über eine oder mehrere dieser Gelenke drehen. Eine eingestellte Position des Endgeräts kann für eine Untersuchung vorteilhaft sein, für den Transport jedoch zu weit über den Wagen hinausragen, wodurch es zu Kollisionen mit beispielsweise einem Türrahmen oder anderen Medizingeräten kommen kann.

Um solche Bewegungen und damit einhergehende etwaige Schäden weitestgehend zu verhindern, kann eine Verriegelung vorgesehen sein, welche es ermöglicht, dass der Federarm oder ein Schwenkgelenk am Ausleger arretiert werden kann. So kann eine Arretierung beispielsweise durch Eingreifen eines Riegels oder eines Hakens in einer Aussparung verwirklicht sein.

### Darstellung der Erfindung

Erfindungsgemäß wurde erkannt, dass derzeit bekannte Lösungen zur Arretierung an einem Ausleger eines Haltesystems für viele Anwendungsfälle ungeeignet sind. Sie lassen beispielsweise keine Arretierung für höhenverstellbare Komponenten, wie Federarme, zu sondern erlauben lediglich eine Arretierung von zwei Auslegern zueinander. Arretierungen im Stand der Technik, die eine solche Fixierung eines Federarms ermöglichen, sind oft umständlich in der Handhabung und erfordern darüber hinaus eine Vielzahl von mechanischen Komponenten, die miteinander intensiv nachbearbeitet werden müssen. So etwa sind regelmäßig separate Drehachsen vorgesehen, welche eine präzise mechanische Bearbeitung erfordern. Eine Vielzahl an mechanischen Komponenten macht weiterhin eine aufwendige Montage erforderlich, welche mit einer längeren Herstellungszeit und erhöhten Herstellungskosten einhergeht.

Darüber hinaus haben bekannte Arretierungen regelmäßig einen hohen Bauraumbedarf, um eine selektive Betätigung der Arretierung zu ermöglichen. Auf diese Weise wird die Montage weiter erschwert. Es kann für die Montage der Arretierung sogar eine teilweise Demontage des Auslegers und/oder des Federarms erforderlich werden. Die Betätigung der Arretierung ist durch die entfernte Anordnung des entsprechenden Tasters von den Arretierungselementen, wobei beispielsweise ein Gestänge zur Übertragung der Bewegung des Tasters vorgesehen sein kann, ebenfalls weniger intuitiv. Die indirekte Betätigung erhöht zudem die Gefahr mechanischer Defekte, weil eine größere Anzahl von Bauteilen verbaut werden muss und jede dieser Komponenten grundsätzlich defektanfällig ist.

Ausgehend von dem bekannten Stand der Technik ist es daher eine Aufgabe der vorliegenden Erfindung, eine verbesserte Vorrichtung zum Verriegeln eines Federarms bereitzustellen, welche insbesondere eine einfachere und kostengünstigere Montage und gleichermaßen eine einfache Bedienung ermöglicht.

Die Aufgabe wird durch den Gegenstand des unabhängigen Anspruchs 1 gelöst. Vorteilhafte Weiterbildungen ergeben sich aus den Unteransprüchen, der Beschreibung und den Figuren.

Entsprechend wird eine Vorrichtung zum Verriegeln eines Federarms vorgeschlagen, umfassend einen ersten Körper zur Befestigung am Federarm und umfassend ein erstes Verriegelungselement, eine Aufnahme zur Befestigung an einer relativ zum Federarm drehbaren Komponente und zum zumindest teilweise Aufnehmen des ersten Körpers, und einen zweiten Körper mit einem zweiten Verriegelungselement, welcher über eine Drehachse des zweiten Körpers relativ zum ersten Körper und zur Aufnahme drehbar angeordnet ist. Weiterhin umfasst die Vorrichtung ein Betätigungselement, welches mit einem ersten Endbereich des zweiten Körpers, welcher sich von der Drehachse und dem zweiten Verriegelungselement wegerstreckt, derart gekoppelt ist, dass das zweite Verriegelungselement im aufgenommenen Zustand des ersten Körpers in der Aufnahme und im nicht betätigten Zustand des Betätigungselements das erste Verriegelungselement zumindest axial begrenzt und im betätigten Zustand des Betätigungselements durch eine Drehung des zweiten Körpers vom ersten Verriegelungselement entkoppelt ist.

Dadurch dass der zweite Körper einen ersten Endbereich aufweist, welcher sich von der Drehachse und dem zweiten Verriegelungselement wegerstreckt, wird erfindungsgemäß eine Kopplung des Betätigungselements mit dem zweiten Körper ermöglicht, ohne dass dafür ein Gestänge für eine Übertragung der Bewegung beziehungsweise Betätigung erforderlich ist. Mit anderen Worten kann der zweite Körper, wie auch das zweite Verriegelungselement als dessen Bestandteil, eine längliche Erstreckung aufweisen, welche eine Kopplung ermöglicht und eine solche Bewegungsübertragung im zweiten Körper integriert. Auf diese Weise kann eine erhebliche Vereinfachung der Montage verwirklicht werden. Denn auf einen Zusammenbau verschiedener mechanischer Komponenten, welche andernfalls für eine solche Übertragung erforderlich wären, kann verzichtet werden.

Weiterhin definiert der zweite Körper seinerseits eine Drehachse. Entsprechend kann auf eine zeitintensive Nachbearbeitung von Drehachsen verzichtet werden. Die Erstreckung bis zum Betätigungselement, einerseits, und die im zweiten Körper integrierte Drehachse, andererseits, ermöglichen darüber hinaus, dass der zweite Körper als Einzelteil ausgebildet sein kann. Bevorzugt ist der zweite Körper als Kunststoffspritzgussteil ausgebildet. Auf diese Weise kann eine hohe Stückzahl bei verhältnismäßig geringen Kosten produktionstechnisch bereitgestellt werden. Gleichermaßen kann eine Vereinfachung der Montage und eine verbesserte mechanische Stabilität bereitgestellt werden.

Der erste Körper und die Aufnahme können weiterhin miteinander so in Eingriff gebracht werden, dass das erste Verriegelungselement und das zweite Verriegelungselement im aufgenommenen Zustand des ersten Körpers in der Aufnahme ineinandergreifen und einrasten beziehungsweise miteinander gekoppelt sind. In diesem Zustand ist der Federarm zumindest axial, also in vertikaler Richtung, zur drehbaren Komponente gesichert. Bevorzugt sind das erste Verriegelungselement und das zweite Verriegelungselement derart ausgebildet, dass zudem eine Sicherung in einer lateralen Richtung beziehungsweise in einer horizontalen Drehrichtung gewährleistet ist.

Alternativ oder zusätzlich kann auch vorgesehen sein, dass eine solche laterale Sicherung oder Fixierung durch die Formgebung des ersten Körpers und der Aufnahme gewährleistet oder zumindest unterstützt wird. So können der erste Körper und die Aufnahme beispielsweise geometrisch aufeinander abgestimmte Oberflächen aufweisen, sodass insbesondere eine formschlüssige Sicherung bereitgestellt werden kann. Entsprechend kann eine (laterale) Drehung des Federarms über eine Drehachse der drehbaren Komponente und relativ zur drehbaren Komponente verhindert werden.

Die drehbare Komponente kann insbesondere ein Ausleger sein. Alternativ kann die drehbare Komponente auch als weiterer Federarm ausgebildet sein, wobei die Vorrichtung beispielsweise in oder an einer Parallelführung des Federarms integriert beziehungsweise angebracht werden kann.

Durch eine Betätigung des Betätigungselements, welches beispielsweise hülsenförmig, hohlzylinderförmig, zylinderförmig oder stiftförmig ausgebildet sein kann, wird weiterhin gewährleistet, dass die Kopplung zwischen dem ersten Verriegelungselement und dem zweiten Verriegelungselement gelöst werden kann. Mit anderen Worten wird durch die erfindungsgemäße Vorrichtung eine einfache lösbare Sicherung des Federarms an der drehbaren Komponente ermöglicht.

Dabei bewirkt die Betätigung des Betätigungselements eine Rotation oder Drehung des zweiten Körpers um die Drehachse des zweiten Körpers. Dadurch dass das Betätigungselement mit dem ersten Endbereich des zweiten Körpers gekoppelt ist, wird vorteilhafterweise eine entsprechende Hebelwirkung bereitgestellt. Denn der erste Endbereich ist zur Drehachse und zum zweiten Verriegelungselement beabstandet angeordnet. Die erfindungsgemäße Ausgestaltung des zweiten Körpers und die Anordnung des Betätigungselements relativ zum zweiten Körper bewirken somit, dass die Verriegelung mit einer geringen Kraft gelöst werden kann. Der zweite Körper ermöglicht hierdurch nicht nur die Verriegelung durch die Kopplung mit dem ersten Verriegelungselement anhand des zweiten Verriegelungselements. Der zweite Körper definiert auch die Drehachse und ermöglicht durch die spezifische Ausgestaltung, dass eine Hebelwirkung bereitgestellt werden kann, ohne dass weitere mechanische Konstruktionen vorgesehen werden müssen.

Das Betätigungselement kann im Wesentlichen senkrecht zum ersten Endbereich angeordnet sein. Alternativ, bevorzugt aber zusätzlich, kann das Betätigungselement im Wesentlichen parallel zur Erstreckungsrichtung des zweiten Verriegelungselements ausgerichtet sein. Durch eine solche Anordnung kann die Vorrichtung besonders kompakt ausgestaltet werden. Das Betätigungselement kann entsprechend neben dem zweiten Verriegelungselement und auf derselben Seite der drehbaren Komponente angeordnet sein und sich, im nicht betätigten Zustand, im Wesentlichen parallel zum zweiten Verriegelungselement erstrecken. Auf diese Weise kann eine Betätigung auf der Seite der Verriegelung bereitgestellt werden kann, wodurch die Betätigung intuitiver und, je nach Dimensionierung der Vorrichtung, sogar einhändig zusammen mit der Bedienung des Federarms erfolgen kann.

Bevorzugt grenzt das Betätigungselement unmittelbar an den ersten Endbereich an. Das Betätigungselement kann den zweiten Körper somit unmittelbar beaufschlagen, sodass auf eine indirekte Übertragung zwischen dem zweiten Körper und dem Betätigungselement verzichtet und die Anzahl der für die Montage der Vorrichtung erforderlichen Bauteile erheblich reduziert werden kann.

Um die axiale Sicherung und bevorzugt auch eine laterale Sicherung des Federarms an der drehbaren Komponente zu unterstützen, können das erste Verriegelungselement und das zweite Verriegelungselement derart ausgebildet sein, dass sie im gekoppelten Zustand formschlüssig ineinandergreifen. Bevorzugt ist das erste Verriegelungselement als Aussparung und das zweite Verriegelungselement hakenförmig ausgebildet. So kann das zweite Verriegelungselement an einem freien Ende beispielsweise einen Haken aufweisen, welcher im gekoppelten Zustand in die Aussparung eingreift und an einer Kante der Aussparung aufliegt, um eine axiale Bewegung des Federarms senkrecht zur Drehrichtung zu verhindern. Mit anderen Worten kann die Aussparung an einer unteren Seite eine Kante aufweisen, welche eine Grenzfläche für die relative Bewegung definiert.

Der Haken weist bevorzugt eine Fase an seiner Oberseite auf. Auf diese Weise kann der Haken beim Einführen des ersten Körpers in die Aufnahme zunächst aus der Aufnahme verdrängt werden, beispielsweise in eine Öffnung oder in eine entsprechende Aussparung in der Aufnahme. Im vollständig aufgenommenen Zustand des ersten Körpers in der Aufnahme kann die Aussparung im ersten Körper dann mit dem Haken ausgerichtet sein und fluchten, sodass der Haken quasi automatisch in der Aufnahme aufgenommen wird. Durch die Fase können entsprechend sowohl das Einführen des ersten Körpers in die Aufnahme als auch das Koppeln des Hakens mit der Aussparung erleichtert werden.

Eine formschlüssige Ausgestaltung kann auch für den ersten Körper und die Aufnahme vorgesehen sein. Obwohl der erste Körper und die Aufnahme verschiedene Formen aufweisen können und beispielsweise auch rechteckig ausgebildet sein können, ist der erste Körper bevorzugt halbkugelförmig und die Aufnahme bevorzugt schalenförmig ausgebildet. Durch diese bevorzugte Ausgestaltung können der erste Körper und die Aufnahme zumindest teilweise ineinander gleiten. Eine Zentrierung des ersten Körpers in der Aufnahme für die Verriegelung kann erheblich erleichtert werden. Denn der erste Körper wird auf diese Weise beim Führen des Federarms parallel zur drehbaren Komponente quasi automatisch in die vorgesehene Position relativ zur drehbaren Komponente geführt und kann an der Oberfläche der Aufnahme zumindest teilweise entlanggleiten.

Weiterhin wird durch eine solche Ausgestaltung auch ein Lösen der Verriegelung erleichtert. Denn beim Lösen der Verriegelung kann eine gleichzeitige Höhenverstellung des Federarms vorgesehen sein, wodurch der Federarm und somit auch der erste Körper relativ zur drehbaren Komponente und der Aufnahme geneigt wird. Die vorteilhaften Rundungen des ersten Körpers und der Aufnahme erleichtern eine solche relative Neigung oder Bewegung. Um die Bewegung oder Höhenverstellung des Federarms relativ zur drehbaren Komponente zu unterstützen, kann ein Vorspannelement in der Aufnahme vorgesehen sein, beispielsweise in Form einer Druckfeder oder eines federnden Druckstücks. Das Vorspannelement kann beispielsweise in einer Öffnung oder Aussparung an der Oberfläche an der Unterseite der Aufnahme vorgesehen sein. Auf diese Weise kann das Entriegeln erleichtert werden. Es muss der erste Körper zum Bedienen des Federarms nur noch die verbleibende Höhe der Aufnahme aus der Aufnahme geschoben werden.

Die Vorrichtung umfasst bevorzugt ein Gehäuse, wobei der zweite Körper im Gehäuse zumindest teilweise aufgenommen und gelagert wird. Das Gehäuse kann entsprechend einen inneren Hohlraum definieren. Das Gehäuse ist weiterhin derart ausgebildet, dass eine dem ersten Körper zugewandte Seite des Gehäuses im montierten Zustand der Vorrichtung von einem angrenzenden Wandbereich der drehbaren Komponente axial begrenzt wird. Das Gehäuse kann entsprechend an einer Stirnseite zumindest teilweise vom angrenzenden Wandbereich überdeckt oder umgeben sein, sodass das Gehäuse in axialer Richtung, das heißt in Richtung des ersten Körpers, begrenzt und vom Wandbereich zurückgehalten wird.

Das Gehäuse kann auf diese Weise in eine Aussparung der drehbaren Komponente eingesetzt und beispielsweise von einer Seite oder der Unterseite der drehbaren Komponente in die Aussparung eingeschoben werden. Der Wandbereich bildet hierfür bevorzugt einen Abschnitt einer tragenden Struktur der drehbaren Komponente.

Die Aufnahme kann sich zumindest teilweise in das Gehäuse hinein erstrecken, wobei die Aufnahme innerhalb des Gehäuses mindestens einen Vorsprung aufweist, welcher von einem Rastelement des Gehäuses vorzugsweise axial begrenzt ist. Die Aufnahme kann beispielsweise einen Zapfen oder ein Bein aufweisen, von dem sich zumindest teilweise ein Vorsprung derart radial erstreckt, dass der Vorsprung in das Rastelement eingreift. Beispielsweise kann der Vorsprung als Rasthaken ausgebildet sein und in eine entsprechende Tasche des Gehäuses eingreifen, um somit eine Bewegung der Aufnahme zumindest in Richtung des ersten Körpers zu verhindern.

Bevorzugt weist die Aufnahme zwei Beine auf, welche sich entlang einer Längserstreckung der Aufnahme erstrecken und an gegenüberliegenden Seiten des zweiten Körpers angeordnet sein können. Jedes Bein weist bevorzugt einen entsprechenden Vorsprung auf. Es können auch auf jeder Seite mehrere Vorsprünge vorgesehen sein, welche in Längsrichtung voneinander beabstandet sind und somit beispielsweise eine Anpassung der axialen Sicherung an der Dimensionierung der drehbaren Komponente oder an Herstellungstoleranzen ermöglichen.

Auf diese Weise kann die Aufnahme einfach von einer dem Gehäuse gegenüberliegenden Seite der drehbaren Komponente in das Gehäuse eingesteckt und am Gehäuse befestigt werden. Die Montage der Vorrichtung kann hierdurch erheblich vereinfacht werden.

Eine besonders vorteilhafte Montage kann weiterhin ermöglicht werden, indem die Aufnahme derart konfiguriert ist, dass sich die Aufnahme im montierten Zustand der Vorrichtung zumindest teilweise über eine Seite eines angrenzenden Wandbereichs der drehbaren Komponente, welche dem ersten Körper zugewandt ist, erstreckt. Beispielsweise kann die Aufnahme eine oder mehrere Rippen aufweisen, welche sich radial von der Aufnahme erstrecken und am angrenzenden Wandbereich, beispielsweise an dessen Oberseite, abgestützt werden. Bevorzugt weisen die Rippen voneinander beabstandete längliche Führungsflächen auf, um die Aufnahme beispielsweise in einer entsprechenden Aussparung in der drehbaren Komponente zu zentrieren.

Durch die Abstützung der Aufnahme an beispielsweise der Oberseite der drehbaren Komponente und die gleichzeitige Abstützung des Gehäuses an der gegenüberliegenden Oberfläche des Wandbereichs kann die Vorrichtung vorteilhaft in die drehbare Komponente eingeklemmt werden. So kann das Gehäuse zunächst in die drehbare Komponente eingesetzt werden; hernach kann die Aufnahme einfach von einer Oberseite in das Gehäuse eingeführt werden und dabei mit dem Gehäuse verrasten. Die Aufnahme, insbesondere die Rippen, wird/werden hierdurch durch die Befestigung im Gehäuseinneren und durch deren Erstreckung über dem äußeren Wandabschnitt axial gehalten. Hierbei wird gleichzeitig bewirkt, dass die axiale Position des Gehäuses beibehalten bleibt. Denn der Wandabschnitt wird durch den oberen Abschnitt der Aufnahme und den unteren Abschnitt des Gehäuses vollständig eingeklemmt.

Zwar kann das Gehäuse alternativ auch mittels Presspassung oder durch Verschrauben in der drehbaren Komponente fixiert werden. Diese Ausgestaltung hat jedoch zudem den Vorteil, dass keine weiteren mechanischen Teile für die Befestigung des Gehäuses und der Aufnahme erforderlich sind. Darüber hinaus kann die Montage durch das einfache Einsetzen und Einschieben des Gehäuses beziehungsweise der Aufnahme und die hierdurch quasi automatisch entstehende Befestigung erheblich erleichtert werden.

Um eine verbesserte Abdichtung zwischen der drehbaren Komponente und der Aufnahme zu ermöglichen, kann sich eine dem ersten Körper zugewandte Seite der Aufnahme im montierten Zustand der Vorrichtung zumindest teilweise über einen angrenzenden Wandbereich der drehbaren Komponente oder über eine Verschalung der drehbaren Komponente hinaus erstrecken.

Beispielsweise können für die drehbare Komponente Verschalungen in Form von ineinandergreifenden Halbschalen vorgesehen sein, welche die tragende Struktur umgeben und eine einfachere Handhabung und Reinigung der drehbaren Komponente ermöglichen. Die Aufnahme kann hierfür an ihrer Oberseite einen Bund definieren, welcher bevorzugt an der Verschalung abschließt. Der Bund kann beispielsweise kreisförmig und bevorzugt konisch ausgebildet sein, sodass ein Übergang zwischen der Aufnahme und der drehbaren Komponente ohne scharfe Kanten bereitgestellt wird und eine Reinigung vereinfacht werden kann.

Gleichermaßen kann das Betätigungselement an dessen Umfang von einer Halterung umgeben und axial begrenzt sein. Die Halterung ist hierbei vorteilhafterweise zumindest teilweise im Gehäuse aufgenommen und weist innerhalb des Gehäuses mindestens einen Vorsprung auf, welcher beispielsweise von einem Rastelement des Gehäuses axial begrenzt ist. Bevorzugt ist an gegenüberliegenden Seiten der Halterung jeweils mindestens ein Vorsprung vorgesehen, beispielsweise in Form eines Rasthakens, welcher beim Einführen der Halterung in das Gehäuse in eine jeweilige Tasche des Gehäuses eingreift. Durch eine beidseitige Anordnung der Vorsprünge kann die Stabilität der axialen Sicherung der Halterung verbessert und das Risiko eines Verkeilens oder seitlichen Kippens der Halterung reduziert werden.

Um das Betätigungselement in der Halterung axial zu begrenzen, kann sich das Betätigungselement an einer dem ersten Endbereich zugewandten Seite radial über ein freies Ende der Halterung erstrecken. Das Betätigungselement kann somit im aufgenommenen Zustand in der Halterung in das Gehäuse eingesetzt werden, wobei eine axiale Bewegung des Betätigungselements vom zweiten Körper und durch die Begrenzung der radialen Erstreckung am Endbereich der Halterung in gegenüberliegenden Richtungen begrenzt ist.

Wie vorstehend für die Aufnahme beschrieben, kann die Halterung, alternativ oder zusätzlich, derart konfiguriert sein, dass sie sich die Halterung im montierten Zustand der Vorrichtung zumindest teilweise über eine Seite eines angrenzenden Wandbereichs der drehbaren Komponente, welche dem ersten Körper zugewandt ist, erstreckt. Entsprechend kann durch die Ausgestaltung der Halterung und des Gehäuses auch eine Klemmbefestigung vorgesehen sein, wodurch die Montage der Vorrichtung erheblich vereinfacht werden kann. Die Halterung kann, wie vorstehend beschrieben, auch eine oder mehrere Rippen aufweisen, welche sich radial von der Halterung wegerstrecken und am angrenzenden Wandbereich, beispielsweise an dessen Oberseite, abgestützt werden; sie weisen bevorzugt voneinander beabstandete längliche Führungsflächen auf, um die Halterung beispielsweise in einer entsprechenden Aussparung in der drehbaren Komponente zu zentrieren.

Auch für die Halterung kann vorgesehen sein, dass sich eine dem ersten Körper zugewandte Seite der Halterung im montierten Zustand der Vorrichtung zumindest teilweise über einen angrenzenden Wandbereich der drehbaren Komponente oder über eine Verschalung der drehbaren Komponente hinaus erstrecken. Entsprechend kann die Halterung auch einen Bund an ihrer Oberseite definieren, welcher eine verbesserte Abdichtung zwischen der drehbaren Komponente und der Halterung bereitstellt. Der Bund kann beispielsweise kreisförmig und bevorzugt konisch ausgebildet sein, sodass ein Übergang zwischen der Halterung und der drehbaren Komponente ohne Auftreten scharfer Kanten bereitgestellt wird; die Reinigung kann somit vereinfacht werden.

Die bevorzugte Ausgestaltung des Gehäuses, der Aufnahme und der Halterung hat nicht nur den Vorteil, dass sie jeweils als Einzelteile mit ihren verschiedenen Funktionen ausgebildet sein können. Auch wird ermöglicht, dass diese jeweils als Kunststoffspritzgussteil ausgebildet sein können. Auf diese Weise kann eine hohe Stückzahl mit verhältnismäßig geringen Kosten produziert werden; schließlich wird hierdurch typischerweise eine Vereinfachung der Montage und eine verbesserte mechanische Stabilität erreicht.

Im Gehäuseinneren kann weiterhin ein Vorspannelement angeordnet sein. Das zweite Verriegelungselement wird im nicht betätigten Zustand des Betätigungselements vom Vorspannelement in einen mit dem ersten Verriegelungselement gekoppelten Zustand vorgespannt. Das Vorspannelement gewährleistet, dass keine versehentliche oder spontane Betätigung des Betätigungselements erfolgen kann und somit eine tatsächliche Betätigung erforderlich ist. Die Entriegelung der axialen Sicherung des Federarms an der drehbaren Komponente wird derart ausschließlich bei einer Betätigung des Betätigungselements ausgelöst. Darüber hinaus kann durch das Vorspannelement ein haptisches Feedback für die Betätigung bereitgestellt und eine für die Entriegelung erforderliche Kraft vorgegeben werden. Der erste Endbereich des zweiten Körpers wird bevorzugt gegen das Betätigungselement gedrückt, wobei das Vorspannelement die zu überwindende Betätigungskraft vorgibt.

Das Vorspannelement ist bevorzugt an einer Unterseite des Gehäuses angeordnet. Bevorzugt ist das Vorspannelement als Druckfeder ausgebildet, wobei die Druckfeder optional an einem Haltedorn am zweiten Körper aufgenommen sein kann. Um eine Hebelwirkung des zweiten Körpers weiter zu verbessern, kann das Vorspannelement an einer Position zwischen dem zweiten Verriegelungselement und dem ersten Endbereich angeordnet sein. Die Position des Vorspannelements befindet sich entsprechend zwischen der Drehachse und dem ersten Endbereich des zweiten Körpers. Der Abstand zum zweiten Verriegelungselement in Längsrichtung des zweiten Körpers beziehungsweise des Gehäuses ist dabei geringer als der Abstand zwischen dem ersten Endbereich und dem Verriegelungselement. Derart wird zwar ein Hebel für das zweite Verriegelungselement bereitgestellt, für das Betätigungselement wird hingegen eine größere Hebelwirkung erreicht. Dadurch können die axiale Sicherung des Federarms unterstützt und gleichzeitig eine leichte Entriegelung bereitgestellt werden.

Der zweite Körper kann weiterhin einen zweiten Endbereich aufweisen, welcher in Längsrichtung vom zweiten Verriegelungselement beabstandet ist und dem ersten Endbereich in Längsrichtung des zweiten Körpers gegenüberliegt, wobei der zweite Endbereich am Gehäuse gelagert ist. Der zweite Endbereich kann derart einen Drehpunkt beziehungsweise die Drehachse des zweiten Körpers bereitstellen. Durch die Lagerung am gegenüberliegenden Endbereich kann die Hebelwirkung für das Betätigungselement und das optionale Vorspannelement vergrößert werden. Darüber hinaus wird durch diese Lagerung eine einfache Form der Lagerung ermöglicht; insbesondere ist die Lagerung vom zweiten Verriegelungselement beabstandet. Somit können für die Lagerung erforderliche Geometrien, sofern erwünscht, spezifisch für den zweiten Endbereich vorgesehen werden. Hierdurch kann beispielsweise die Komplexität des zweiten Körpers und der Aufnahme reduziert werden.

Um die Lagerung und die Drehung des zweiten Körpers zu unterstützen, kann der zweite Endbereich zwei sich senkrecht zur Längsrichtung des zweiten Körpers erstreckende Zapfen aufweisen, welche die Drehachse bilden und durch Aussparungen in der Aufnahme axial begrenzt sind. Die Zapfen können beispielsweise gerundet oder zylindrisch ausgebildet sein und sich an beiden Seiten des Körpers befinden, wodurch insbesondere eine gleichmäßige Drehung des zweiten Körpers bei einer gleichmäßigen Abstützung beziehungsweise Lagerung ermöglicht wird.

Um den zweiten Endbereich axial zu begrenzen, kann die Aufnahme weiterhin zwei Beine aufweisen, welche in das Gehäuse hineinragen und eine Aussparung für den jeweiligen Zapfen aufweisen, um den jeweiligen Zapfen an dessen Oberseite aufzunehmen und zu begrenzen. Die Aussparungen können insbesondere halbkreisförmig ausgebildet sein und somit eine Öffnung zum Aufnehmen der Zapfen aufweisen. Auf diese Weise kann der zweite Körper zunächst im Gehäuse platziert und das Gehäuse anschließend in die drehbare Komponente eingesetzt werden. Hernach kann die Aufnahme einfach von der Oberseite der drehbaren Komponente in eine Aussparung eingeführt und auf die Zapfen aufgesetzt werden. Hierdurch wird die Aufnahme bevorzugt gleichzeitig durch die vorstehend beschriebenen Rastelemente und Vorsprünge am Gehäuse fixiert.

Die Beine können sich beispielsweise auf der Unterseite des Gehäuses abstützen. Die Beine können beispielsweise für die bevorzugten Vorsprünge zur axialen Sicherung der Aufnahme oder separat vorgesehen sein. Alternativ können im Gehäuse entsprechende Aufnahmen vorgesehen sein, welche insbesondere halbkreisförmig ausgebildet sein können, sodass die Zapfen am vollständigen Umfang vom Gehäuse und der Aufnahme gehalten werden, eine Drehung des zweiten Körpers jedoch nicht beeinträchtigt wird.

Die erfindungsgemäße Vorrichtung kann insbesondere in einem Haltesystem für ein Endgerät implementiert sein. So kann ein Haltesystem beispielsweise einen Federarm aufweisen, welcher mit einem Ausleger oder einer anderen drehbaren Komponente gekoppelt ist, wobei die Vorrichtung am Ausleger und am Federarm befestigt beziehungsweise darin integriert ist. Auf diese Weise kann durch die Vorrichtung eine besonders einfache Verriegelung und Entriegelung des Federarms relativ zum Ausleger bereitgestellt werden. Die Montage der Vorrichtung kann gegenüber herkömmlichen Arretiervorrichtungen erheblich vereinfacht werden.

Bevorzugt weist die Vorrichtung ein Gehäuse auf, wie vorstehend beschrieben. Das Gehäuse kann insbesondere in einer Aussparung der drehbaren Komponente eingesetzt sein. Bevorzugt wird das Gehäuse mittels eines Formschlusses mit der Aufnahme und der bevorzugten Halterung in der Aussparung der drehbaren Komponente gehalten. Auf diese Weise kann beispielsweise auf eine Verschraubung des Gehäuses verzichtet werden, wodurch die Montage der Vorrichtung erleichtert und die strukturelle Stabilität der drehbaren Komponente verbessert werden können.

Wie vorstehend beschrieben, können zumindest die Aufnahme, der erste Körper, der zweite Körper und das Betätigungselement, bevorzugt aber auch das optionale Gehäuse und die optionale Halterung, als Kunststoffspritzgussteile ausgebildet sein. Derart werden zur Montage keine weiteren Einzelteile, wie ein Gestänge, Sicherungsringe oder Sicherungsscheiben, benötigt. Auch kann auf eine komplexe Nachbearbeitung verzichtet werden.

Gleichermaßen können die zur Befestigung der Vorrichtung bevorzugten Geometrien am Federarm und an der drehbaren Komponente, insbesondere in Form eines Auslegers, vorteilhafterweise in einer entsprechend tragenden Struktur mit eingegossen werden, wenn die tragende Struktur als Gussteil ausgeführt wird. Auf diese Weise kann die erfindungsgemäße Vorrichtung auf besonders einfache Weise an der drehbaren Komponente und am Federarm befestigt werden.

### Kurze Beschreibung der Figuren

Bevorzugte weitere Ausführungsformen der Erfindung werden durch die nachfolgende Beschreibung der Figuren näher erläutert. Es zeigen:
Figur 1 einen Längsschnitt einer erfindungsgemäßen Vorrichtung in einem Haltesystem;
Figur 2 eine perspektivische Darstellung des zweiten Körpers gemäß Figur 1;
Figur 3 eine perspektivische Darstellung des zweiten Körpers in einem Gehäuse gemäß Figur 1;
Figur 4 eine perspektivische Darstellung des Gehäuses gemäß Figur 1 ohne den zweiten Körper;
Figur 5 eine seitliche perspektivische Darstellung des ersten Körpers gemäß Figur 1;
Figur 6 eine obere perspektivische Darstellung des ersten Körpers gemäß Figur 5;
Figur 7 eine untere perspektivische Darstellung der Aufnahme gemäß Figur 1;
Figur 8 eine Draufsicht der Aufnahme gemäß Figur 7;
Figur 9 eine perspektivische Schnittdarstellung der Vorrichtung gemäß Figur 1 am zweiten Endbereich;
Figur 10 eine perspektivische Schnittdarstellung der Vorrichtung gemäß Figur 1 am ersten Endbereich;
Figur 11 eine untere perspektivische Darstellung der Halterung gemäß Figur 1;
Figur 12 einen schematischen Längsschnitt der Halterung gemäß Figur 11; und
Figur 13 eine untere perspektivische Darstellung des Betätigungselements gemäß Figur 1.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Auf eine wiederholte Beschreibung dieser Elemente wird teilweise verzichtet, um Wiederholungen zu vermeiden.

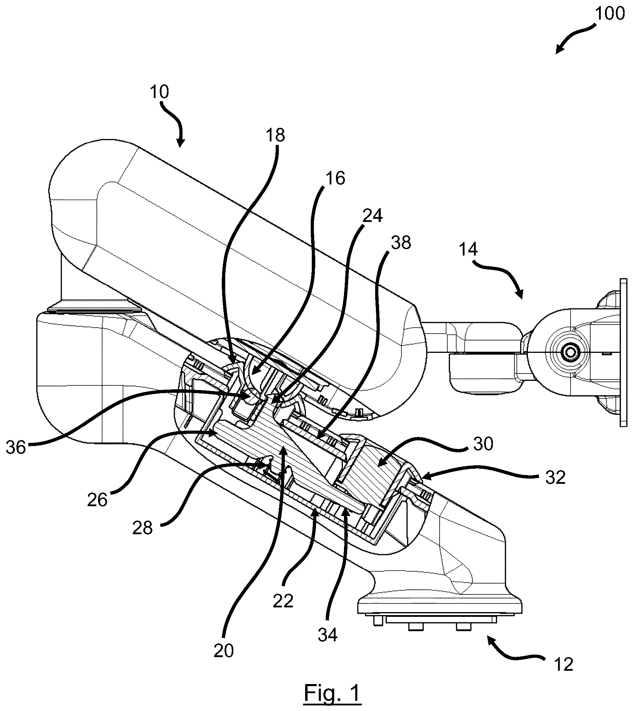

In Figur 1 ist ein beispielhaftes Haltesystem 100 für ein Endgerät gezeigt, wobei das Haltesystem 100 einen drehbaren und höhenverstellbaren Federarm 10 aufweist, welcher mit einer drehbaren Komponente 12 über eine entsprechende Drehachse gekoppelt ist. Im vorliegenden Beispiel ist die drehbare Komponente 12 als Ausleger ausgebildet, welche beispielsweise auf einem Wagen montiert werden kann. Am anderen Ende des Federarms 10 ist weiterhin ein Schwenkgelenk 14 für das Endgerät vorgesehen. Das Endgerät kann beispielsweise ein Monitor sein und anhand des Schwenkgelenks 14 sowohl gedreht und geneigt und optional seitlich gekippt werden.

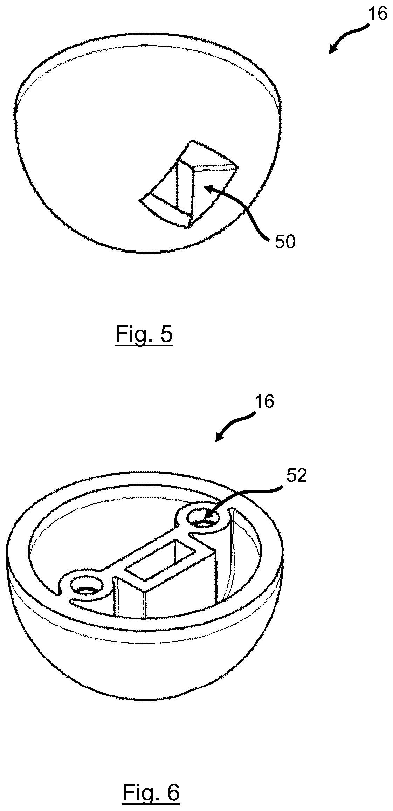

Die erfindungsgemäße Vorrichtung ist in der gezeigten Ausführungsform am Federarm 10 und an der drehbaren Komponente 12 befestigt. Der erste Körper 16 ist halbkugelförmig ausgebildet und an der Unterseite des Federarms 10 verschraubt. Die Aufnahme 18 ist entsprechend halbschalenförmig ausgebildet und dimensioniert, um den ersten Körper 16 aufzunehmen. Um den Federarm 10 an der drehbaren Komponente 12 zu arretieren, weist die Vorrichtung weiterhin einen zweiten Körper 20 auf, welcher eine längliche Form aufweist und in einem Gehäuse 22 der Vorrichtung angeordnet ist. Zum Verriegeln des Federarms 10 weist der zweite Körper 20 ein zweites Verriegelungselement 24 auf, welches in ein erstes Verriegelungselement des ersten Körpers 16 eingreift und den Federarm 10 somit axial an der drehbaren Komponente 12 sichert. Das zweite Verriegelungselement 24 ist hakenförmig ausgebildet und kann auf diese Weise besonders vorteilhaft in eine Aussparung des ersten Körpers 16 eingreifen, welche in Figur 5 detaillierter gezeigt ist.

Um den Federarm 10 im verriegelten Zustand zu behalten, ist der zweite Körper 20 in eine entsprechende Rastposition vorgespannt. Der zweite Körper 20 ist dabei derart ausgebildet, dass er eine Drehachse an einem zweiten Endbereich 26 des zweiten Körpers 20 definiert. Der zweite Endbereich 26 ist vom zweiten Verriegelungselement 24 in Längsrichtung beabstandet und ermöglicht somit, dass das zweite Verriegelungselement 24 zwischen einer Rastposition und einer freien, entriegelten Position gedreht werden kann. Die Vorspannung in die Rastposition wird dabei von einem Vorspannelement 28 unterstützt, welches an der Unterseite des Gehäuses 22 angeordnet ist und den zweiten Körper 20 beaufschlagt. Bevorzugt ist das Vorspannelement 28 als Druckfeder ausgebildet, wobei die Druckfeder von einem Haltedorn des zweiten Körpers 20 aufgenommen sein kann.

Eine Bewegung in die entriegelte Position wird durch die Betätigung eines Betätigungselements 30 ausgelöst, welches von einer Halterung 32 umgeben und dadurch axial relativ zum Gehäuse 22 gesichert ist. Das Betätigungselement 30 ist auf Höhe eines ersten Endbereichs 34 des zweiten Körpers 20 vorgesehen und ist derart angeordnet, dass das Betätigungselement 30 unmittelbar an den ersten Endbereich 34 angrenzt. Der erste Endbereich 34 liegt dabei weiter entfernt vom zweiten Verriegelungselement 24 als das Vorspannelement 28, sodass durch eine Betätigung des Betätigungselements 30 und im Vergleich zum Vorspannelement 28 eine verhältnismäßig größere Hebelwirkung am zweiten Körper 20 bereitgestellt wird und das zweite Verriegelungselement 24 entsprechend in die entriegelte Position bewegt werden kann.

Um die Entriegelung zu erleichtern, ist ein optionales weiteres Vorspannelement 36 vorgesehen, welches in einer Öffnung an der Unterseite der Aufnahme 18 vorgesehen sein kann und den ersten Körper 16 bei Betätigung des Betätigungselements 30 aus der Aufnahme 18 nach oben drückt.

In der Figur 1 ist weiterhin ein Wandbereich 38 der drehbaren Komponente 12 gezeigt. Das Gehäuse 22 ist dabei derart in die drehbare Komponente 12 eingesetzt, beispielsweise in einer entsprechenden seitlichen Aussparung, dass der obere Randbereich des Gehäuses 22 an die Unterseite des Wandbereichs 38 grenzt. Der Wandbereich 38 kann beispielsweise Teil einer tragenden Struktur der drehbaren Komponente 12 sein und somit eine gewisse strukturelle Festigkeit aufweisen, um das Gehäuse 22 axial nach oben zu sichern.

Auf der gegenüberliegenden, oberen Seite des Wandbereichs 38 sind Abschnitte der Aufnahme 18 und der Halterung 32 vorgesehen, welche sich am Wandbereich 38 abstützen. Auf diese Weise können das Gehäuse 22, die Aufnahme 18 und die Halterung 32 vorteilhafterweise eingeklemmt werden, indem die Aufnahme 18 und die Halterung 32 zudem im Gehäuseinneren am Gehäuse 22 befestigt sind. Mit anderen Worten wird der Wandbereich 38 derart von der unteren Seite vom Gehäuse 22 und von der oberen Seite von der Aufnahme 18 und der Halterung 32 angedrückt beziehungsweise zusammengedrückt. Hierdurch kann eine Montage auf einfache Weise erfolgen, indem die Halterung 32 und die Aufnahme 18 umstandslos in das Gehäuse 22 eingeführt werden, nachdem das Gehäuse 22 zunächst in die drehbare Komponente 12 eingesetzt worden ist. Denn durch das Einführen der Aufnahme 18 und der Halterung 32 werden diese quasi automatisch im Gehäuseinneren verrastet, sodass die entsprechende Klemmkraft und Fixierung am Wandbereich 38 bereitgestellt wird.

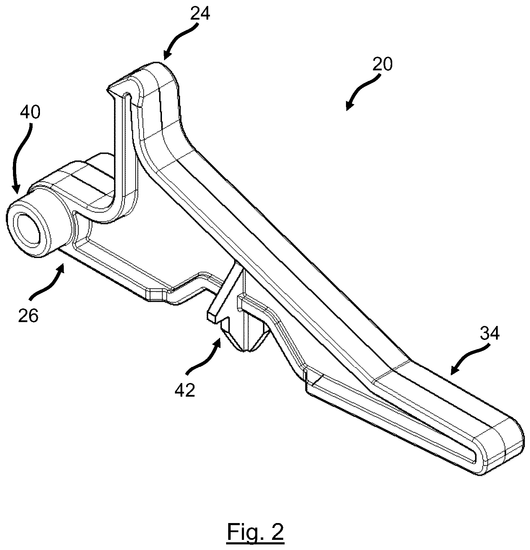

In Figur 2 ist die längliche Ausgestaltung des zweiten Körpers 20 detaillierter gezeigt. Zu sehen ist, dass sich am zweiten Endbereich 26 an gegenüberliegenden Seiten des zweiten Körpers 20 zwei Zapfen 40 im Wesentlichen senkrecht zum zweiten Körper 20 erstrecken. Die Zapfen 40 bilden dabei die Drehachse des zweiten Körpers 20, sodass die Drehachse vorteilhafterweise vollständig im zweiten Körper 20 integriert ist. In Richtung des ersten Endbereichs 34 ist weiterhin ein Haltedorn 42 vorgesehen, an dem ein Druckfeder zum Vorspannen des zweiten Verriegelungselements 24 aufgenommen sein kann.

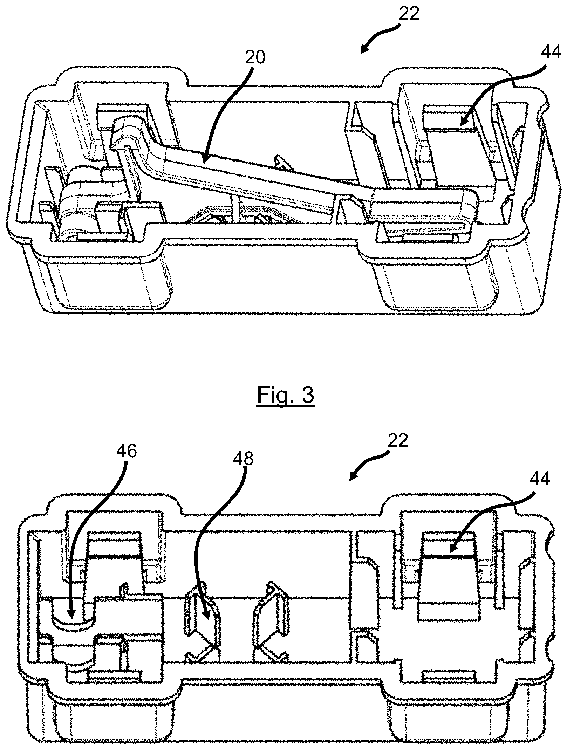

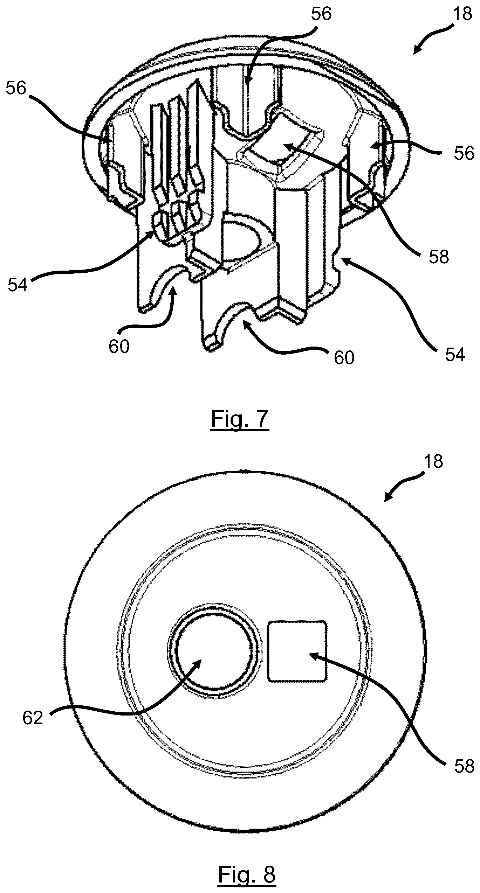

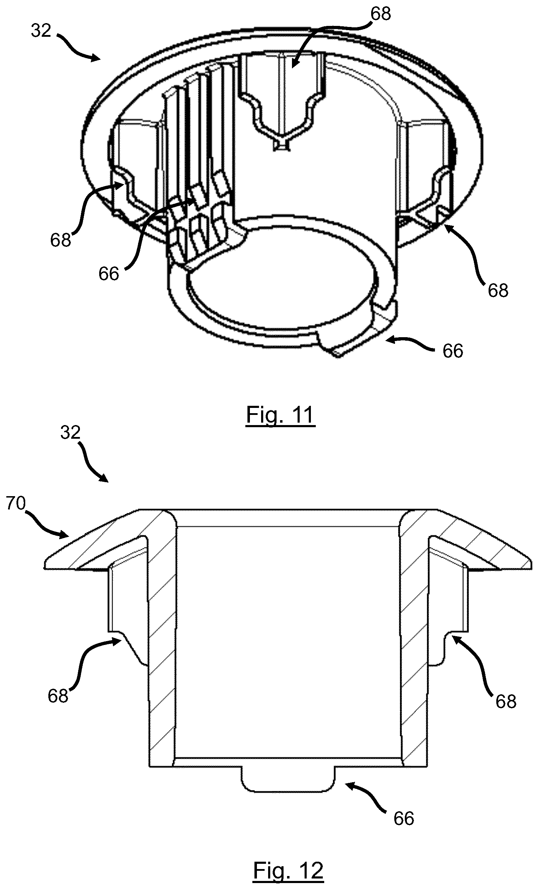

In den Figuren 3 und 4 ist das Gehäuse 22 mit dem zweiten Körper 20 (Figur 3) und ohne den zweiten Körper 20 (Figur 4) dargestellt. In diesen Figuren ist der obere Randbereich, welcher an den Wandbereich 38 der drehbaren Komponente 12 angrenzt, deutlicher dargestellt. Weiterhin sind in diesen Figuren auf Höhe jedes Endbereichs 26,34 des zweiten Körpers 20 jeweils zwei Rastelemente 44 in Form von Taschen zu sehen. An diesen Taschen können entsprechende Vorsprünge der Aufnahme 18 beziehungsweise der Halterung 32 aufgenommen werden, wie in den Figuren 7 und 11 detaillierter gezeigt.

Auf Höhe des zweiten Endbereichs 26 sind weiterhin zwei halbkreisförmige Aussparungen 46 vorgesehen, in die die Zapfen 40 des zweiten Körpers 20 aufgenommen werden. Ebenfalls sind Führungsrippen 48 auf Höhe des Haltedorns 42 beziehungsweise des Vorspannelements 28 vorgesehen. Die Führungsrippen 48 können sowohl zur Zentrierung des zweiten Körpers 20 als auch zur Aufnahme des Haltedorns 42 beziehungsweise des Vorspannelements 28 dienen.

In den Figuren 5 und 6 ist der erste Körper 16 in verschiedenen Ansichten dargestellt, wobei das erste Verriegelungselement 50 vorliegend als Aussparung ausgebildet ist. Die Aussparung bildet somit einerseits eine Kante für den Haken des zweiten Verriegelungselements 24. Hierdurch kann der Federarm 10 zumindest axial an der drehbaren Komponente 12 gesichert werden. Die Aussparung ist auch derart ausgebildet, dass eine laterale oder seitliche Bewegung des ersten Körpers 16 aufgrund der formschlüssigen Aufnahme des Hakens verhindert wird. Eine Bewegung in die entriegelte Position wird jedoch ermöglicht.

In der oberen perspektivischen Ansicht in Figur 6 sind weiterhin zwei Bohrungen 52 gezeigt, mittels welcher eine Befestigung des ersten Körpers 16 am Federarm 10 bereitgestellt werden kann. Die Bohrungen 52 können beispielsweise ein Innengewinde aufweisen, wodurch der erste Körper 16 einfach am Federarm 10 verschraubt werden kann.

In den Figuren 7 und 8 ist die Aufnahme 18 in verschiedenen Ansichten dargestellt. Wie in Figur 7 gezeigt, weist die Aufnahme voneinander beabstandete Beine mit endseitigen Vorsprüngen 54 auf. Die Vorsprünge 54 sind dazu konfiguriert, mit den in den Figuren 3 und 4 gezeigten Rastelementen 44 beziehungsweise Taschen auf Höhe des zweiten Endbereichs 26 zu verrasten. Um die Vorsprünge 54 mit den Rastelementen 44 in Eingriff zu bringen, weisen die Vorsprünge 54 jeweilige Fasen auf, wodurch sie beim Einführen in das Gehäuse 22 in die verrastete Position vorgespannt werden.

Am oberen Randbereich der Aufnahme 18 sind weiterhin voneinander beabstandete Rippen 56 vorgesehen, welche sich zumindest teilweise radial nach außen erstrecken. Die Rippen 56 dienen als Stützflächen an der Oberseite des in der Figur 1 gezeigten Wandbereichs 38, sodass die Aufnahme 18 axial am Wandbereich 38 beziehungsweise an der drehbaren Komponente 12 gesichert werden kann. Die Rippen 56 sind weiterhin derart bemessen, dass sie eine Zentrierung der Aufnahme 18 in einer entsprechenden Öffnung oder Aussparung im Wandbereich 38 ermöglichen, sodass die Aufnahme 18 beim Einführen in das Gehäuse 22 in eine vorgegebene Position relativ zum Gehäuse 22 und zum ersten Körper 16 ausgerichtet werden kann.

An der Unterseite der Aufnahme sind weiterhin zwei Aussparungen 60 vorgesehen, welche halbkreisförmig ausgebildet sind und jeweils an einem Stützbein ausgebildet sind. Durch diese Aussparungen 60 können die Oberseite der in der Figur 2 gezeigten Zapfen 40 aufgenommen und die Zapfen 40 beziehungsweise die Drehachse des zweiten Körpers axial gesichert werden.

An der dem ersten Körper 16 zugewandten Seite der Aufnahme 18 sind weiterhin eine Aussparung 58 sowie eine Öffnung 62 vorgesehen. Die Aussparung 58 dient zur Führung des zweiten Verriegelungselements 24 vom Gehäuseinneren zum zweiten Verriegelungselement des ersten Körpers 16. In der Öffnung 62 kann vorteilhafterweise ein weiteres Vorspannelement 36 aufgenommen sein, wie in Figur 1 gezeigt.

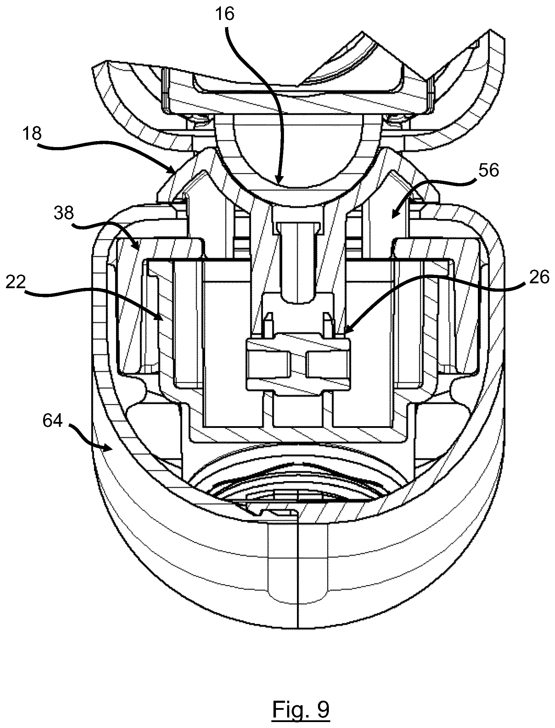

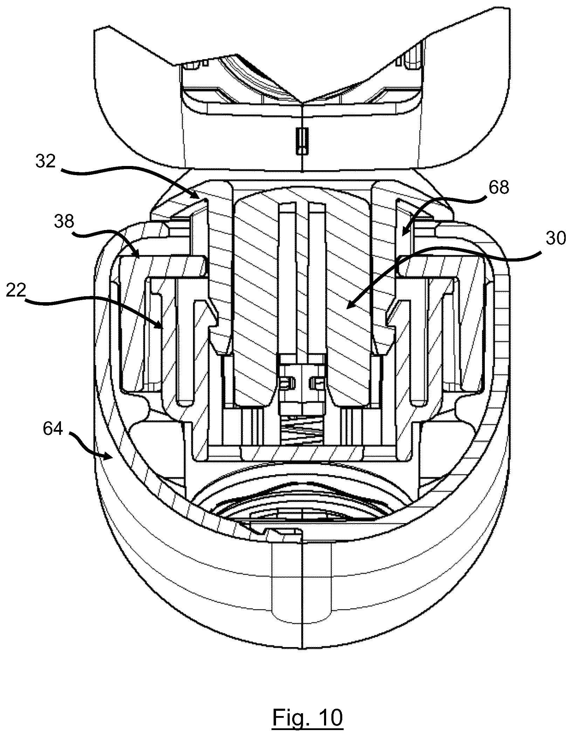

In den Figuren 9 und 10 sind perspektivische Schnittdarstellungen der erfindungsgemäßen Vorrichtung am zweiten Endbereich 26 beziehungsweise am ersten Endbereich 34 gezeigt. In Figur 9 ist detailliert gezeigt, dass die Aufnahme 18 den ersten Körper 16 nahezu vollständig aufnehmen kann. Weiterhin geht hieraus eindeutig hervor, wie sich sowohl die Aufnahme 18 als auch das Gehäuse 22 am Wandbereich 38 an gegenüberliegenden Seiten abstützen. So liegen die Rippen 56 der Aufnahme 18 an der Oberseite des Wandbereichs 38, welcher eine Aussparung definiert, auf und es stößt der obere Randbereich des Gehäuses 22 ebenfalls an der Unterseite des Wandbereichs 38 an. Auf ähnliche Weise liegen die Rippen 68 der Halterung 32 an der Oberseite des Wandbereichs 38 auf, wie in Figur 10 gezeigt.

Weiterhin ist eine Verschalung 64 in den Figuren 9 und 10 dargestellt, welche die tragende Struktur der drehbaren Komponente 12 umgibt und eine im Wesentlichen durchgehende Oberfläche definiert. Um scharfe Kanten oder Stufen zwischen der Aufnahme 18 und der Verschalung 64 zu vermeiden, weist die Aufnahme 18 einen radialen Bund auf, welcher konisch ausgebildet ist und mit dem oberen Randbereich der Verschalung 64 fluchtet.

In Figur 10 ist die entsprechende Anordnung der Halterung 32 am ersten Endbereich 34 gezeigt. Zu sehen ist, dass das Betätigungselement 30 in der Halterung 32 aufgenommen ist und am unteren Ende eine radiale, seitliche Erstreckung aufweist. Diese wird vom unteren Ende der Halterung 32 aufgenommen und stellt somit eine axiale Sicherung des Betätigungselements 30 relativ zur Halterung 32 bereit.

In den Figuren 11 bis 13 sind die Halterung 32 und das Betätigungselement 30 detaillierter gezeigt. So sind in den Figuren 11 und 12 ebenfalls Vorsprünge 66 und Rippen 68 dargestellt, welche, ähnlich wie die Vorsprünge 54 und Rippen 56 der Aufnahme 18, eine axiale Sicherung beziehungsweise Abstützung und Zentrierung der Halterung 32 ermöglichen. Die Vorsprünge 66 greifen dabei in die Rastelemente 44 des Gehäuses 22 am ersten Endbereich 34 ein. Die Rastelelemente sind beispielsweise in den Figuren 3 und 4 gezeigt. Die Vorsprünge 66 können, wie in Figur 11 gezeigt, an der Wand, welche das Betätigungselement 30 aufnimmt, vorgesehen und optional darin integriert sein. Die Rippen 68 liegen weiterhin an der Oberseite des Wandbereichs 38, wie in Figur 10 gezeigt.

Die Halterung 32 weist weiterhin einen inneren Durchgang oder Hohlraum auf, in dem das Betätigungselement 30 aufgenommen wird, wie sich aus Figur 11 und insbesondere aus Figur 12 ergibt. In Figur 12 ist weiterhin der obere Bund 70 detailliert dargestellt, welcher eine vorteilhafte Abdichtung der Halterung 32 zur Verschalung 64 ermöglicht.

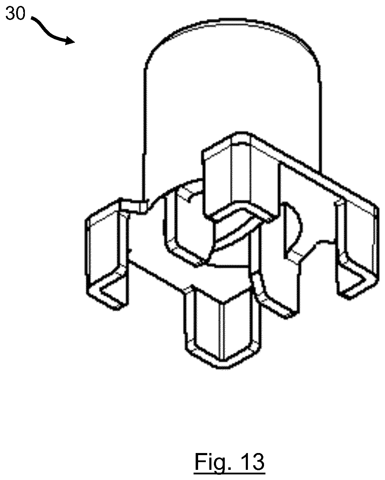

In Figur 13 ist das Betätigungselement 30 in einer unteren perspektivischen Detailansicht gezeigt. Das Betätigungselement 30 ist im Wesentlichen zylindrisch ausgebildet, kann jedoch alternativ auch eine andere Form aufweisen, beispielsweise eine rechteckige, ellipsoide oder polygonale Form. Am unteren Bereich des Betätigungselements sind Führungsrippen vorgesehen, welche mit optionalen Führungsrippen am ersten Endbereich 34 am Gehäuse 22 ausgerichtet sein können. Auf diese Weise kann das Betätigungselement 30 auf einfache Weise in eine vorgegebene Position zentriert werden. Am unteren Bereich des Betätigungselements 30 ist weiterhin eine mittige Aufnahme für den ersten Endbereich 34 des zweiten Körpers 20 vorgesehen, sodass das Betätigungselement 30 unmittelbar mit dem zweiten Körper 20 gekoppelt werden kann. Die Aufnahme des Betätigungselements 30 kann dabei sowohl an die Geometrie des ersten Endbereichs 34 als auch an die Ausrichtung des zweiten Verriegelungselements 24 angepasst sein, um derart eine im Wesentlichen parallele Ausrichtung des Betätigungselements 30 zum zweiten Verriegelungselement 24 zu ermöglichen, welche bevorzugt senkrecht zur oberen Oberfläche der drehbaren Komponente 12 ausgerichtet ist.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 10: Federarm
- 12: drehbare Komponente
- 14: Schwenkgelenk
- 16: erster Körper
- 18: Aufnahme
- 20: zweiter Körper
- 22: Gehäuse
- 24: zweites Verriegelungselement
- 26: zweiter Endbereich
- 28: Vorspannelement (Gehäuse 22)
- 30: Betätigungselement
- 32: Halterung
- 34: erster Endbereich
- 36: Weiteres Vorspannelement (Aufnahme 18)
- 38: Wandbereich
- 40: Zapfen
- 42: Haltedorn
- 44: Rastelement
- 46: Aussparung
- 48: Führungsrippe
- 50: erstes Verriegelungselement
- 52: Bohrung
- 53: Nut
- 54: Vorsprung (Aufnahme 18)
- 56: Rippe (Aufnahme 18)
- 58: Aussparung (Aufnahme 18, oben)
- 60: Aussparung (Aufnahme 18, unten)
- 62: Öffnung
- 64: Verschalung
- 66: Vorsprung (Halterung 32)
- 68: Rippe (Halterung 32)
- 70: Bund

- 100: Haltesystem

## Patentansprüche

1. Vorrichtung zum Verriegeln eines Federarms (10), umfassend
- einen ersten Körper (16) zur Befestigung am Federarm (10), umfassend ein erstes Verriegelungselement (50),
- eine Aufnahme (18) zur Befestigung an einer relativ zum Federarm (10) drehbaren Komponente (12) und zum zumindest teilweise Aufnehmen des ersten Körpers (16),
- einen zweiten Körper (20) mit einem zweiten Verriegelungselement (24), welcher über eine Drehachse des zweiten Körpers (20) relativ zum ersten Körper (16) und zur Aufnahme (18) drehbar angeordnet ist, und
- ein Betätigungselement (30), welches mit einem ersten Endbereich (34) des zweiten Körpers (20), welcher sich von der Drehachse und dem zweiten Verriegelungselement (24) wegerstreckt, derart gekoppelt ist, dass das zweite Verriegelungselement (24) im aufgenommenen Zustand des ersten Körpers (16) in der Aufnahme (18) und im nicht betätigten Zustand des Betätigungselements (30) das erste Verriegelungselement (50) zumindest axial begrenzt und das zweite Verriegelungselement (24) im betätigten Zustand des Betätigungselements (30) durch eine Drehung des zweiten Körpers (20) vom ersten Verriegelungselement (50) entkoppelt ist.

2. Vorrichtung gemäß Anspruch 1, wobei das Betätigungselement (30) im Wesentlichen senkrecht zum ersten Endbereich (34) angeordnet ist und/oder wobei das Betätigungselement (30) im Wesentlichen parallel zur Erstreckungsrichtung des zweiten Verriegelungselements (24) ausgerichtet ist.

3. Vorrichtung gemäß einem der vorstehenden Ansprüche, wobei das Betätigungselement (30) unmittelbar an den ersten Endbereich (34) angrenzt.

4. Vorrichtung gemäß einem der vorstehenden Ansprüche, wobei das erste Verriegelungselement (50) und das zweite Verriegelungselement (24) im gekoppelten Zustand formschlüssig ineinandergreifen.

5. Vorrichtung gemäß einem der vorstehenden Ansprüche, wobei das erste Verriegelungselement (50) als Aussparung und das zweite Verriegelungselement (24) hakenförmig ausgebildet sind.

6. Vorrichtung gemäß einem der vorstehenden Ansprüche, wobei der erste Körper (16) halbkugelförmig und die Aufnahme (18) schalenförmig ausgebildet sind.

7. Vorrichtung gemäß einem der vorstehenden Ansprüche, umfassend ein Gehäuse (22) zum zumindest teilweise Aufnehmen und Lagern des zweiten Körpers (20), wobei das Gehäuse (22) derart ausgebildet ist, dass eine dem ersten Körper (16) zugewandte Seite des Gehäuses (22) im montierten Zustand der Vorrichtung von einem angrenzenden Wandbereich (38) der drehbaren Komponente (12) axial begrenzt wird.

8. Vorrichtung gemäß Anspruch 7, wobei die Aufnahme (18) sich zumindest teilweise in das Gehäuse (22) hinein erstreckt und wobei die Aufnahme (18) innerhalb des Gehäuses (22) mindestens einen Vorsprung (54) aufweist, welcher von einem Rastelement (44) des Gehäuses (22) axial begrenzt ist.

9. Vorrichtung gemäß Anspruch 8, wobei die Aufnahme (18) derart konfiguriert ist, dass die Aufnahme (18) sich im montierten Zustand der Vorrichtung zumindest teilweise über eine Seite eines angrenzenden Wandbereichs (38) der drehbaren Komponente (12), welche dem ersten Körper (16) zugewandt ist, erstreckt.

10. Vorrichtung gemäß einem der Ansprüche 7 bis 9, wobei das Betätigungselement (30) an dessen Umfang von einer Halterung (32) umgeben und axial begrenzt ist, wobei die Halterung (32) zumindest teilweise im Gehäuse (22) aufgenommen ist und wobei die Halterung (32) innerhalb des Gehäuses (22) mindestens einen Vorsprung (66) aufweist, welcher von einem Rastelement (44) des Gehäuses (22) axial begrenzt ist.

11. Vorrichtung gemäß Anspruch 10, wobei die Halterung (32) derart konfiguriert ist, dass sich die Halterung (32) im montierten Zustand der Vorrichtung zumindest teilweise über eine Seite eines angrenzenden Wandbereichs (38) der drehbaren Komponente (12), welche dem ersten Körper (16) zugewandt ist, erstreckt.

12. Vorrichtung gemäß einem der Ansprüche 7 bis 11, wobei im Gehäuseinneren ein Vorspannelement (28) angeordnet ist, wobei das zweite Verriegelungselement (24) im nicht betätigten Zustand des Betätigungselements (30) vom Vorspannelement (28) in den gekoppelten Zustand mit dem ersten Verriegelungselement (50) vorgespannt ist.

13. Vorrichtung gemäß Anspruch 12, wobei das Vorspannelement (28) an einer Position zwischen dem zweiten Verriegelungselement (24) und dem ersten Endbereich (34) angeordnet ist.

14. Vorrichtung gemäß einem der Ansprüche 7 bis 13, wobei der zweite Körper (20) einen zweiten Endbereich (26) aufweist, welcher in Längsrichtung vom zweiten Verriegelungselement (24) beabstandet ist und dem ersten Endbereich (34) in Längsrichtung des zweiten Körpers (20) gegenüberliegt, wobei der zweite Endbereich (26) am Gehäuse (22) gelagert ist.

15. Vorrichtung gemäß Anspruch 14, wobei der zweite Endbereich (26) zwei sich senkrecht zur Längsrichtung des zweiten Körpers (20) erstreckende Zapfen (40) aufweist, welche die Drehachse bilden und durch Aussparungen (60) in der Aufnahme (18) und in dem Gehäuse (22) axial begrenzt sind.

16. Haltesystem (100) für eine Vorrichtung, umfassend einen Federarm (10) und einer damit drehbar gekoppelten drehbaren Komponente (12), bevorzugt einem Ausleger, und eine Vorrichtung gemäß einem der vorstehenden Ansprüche, wobei der erste Körper am Federarm (10) und die Aufnahme an der drehbaren Komponente (12) befestigt sind.

17. Haltesystem (100) gemäß Anspruch 16, wobei die Vorrichtung eine Vorrichtung gemäß einem der Ansprüche 7 bis 15 ist und wobei das Gehäuse (22) in einer Aussparung der drehbaren Komponente (12) eingesetzt ist.
